# EUROPEAN PATENT SPECIFICATION

(11) **EP 2 594 310 B1**
(45) Date of publication and mention of the grant of the patent: **02.03.2016**
(21) Application number: 12193068.9
(22) Date of filing: 16.11.2012
(51) Int. Cl.: A61M 25/10, A61B 1/00

(54) **Applicator for resilient ring device**
Applikator für eine elastische Ringvorrichtung
Applicateur pour dispositif de bague élastique

(30) Priority: 18.11.2011 JP 2011252272; 09.10.2012 JP 2012224189
(43) Date of publication of application: 22.05.2013
(73) Proprietor: Fujifilm Corporation, Minato-ku Tokyo (JP)
(72) Inventor: Yoshimoto, Yoshiyuki, Ashigarakami-gun, Kanagawa (JP); Iida, Tomokazu, Ashigarakami-gun, Kanagawa (JP)
(74) Representative: Klunker . Schmitt-Nilson . Hirsch

(56) References cited:
- JP-A- 2009 160 093
- US-A1- 2010 305 404

## Description

### BACKGROUND OF THE INVENTION

### 1. Field of the Invention

The preset invention relates to an applicator for fitting a resilient ring device according to the preamble of claim 1. More particularly, the present invention relates to an applicator capable of fitting a resilient ring device such as a rubber ring or balloon on an endoscope or other medical instrument in a rapid and exact manner.

### 2. Description Related to the Prior Art

Various uses of a balloon with an endoscope are known. An example of the endoscope is one for diagnosing small or large intestines. The balloon of an expandable form is mounted on an elongated tube of the endoscope. Also, the balloon may be mounted on a medical assist device for the elongated tube, such as a sliding tube or overtube. The elongated tube or the medical assist device is anchored on a wall of a body cavity by expansion of the balloon. Also, an ultrasonic diagnosis system is known, and includes an ultrasonic probe. The balloon of an expandable form is used to surround an ultrasonic scanning unit in the probe, and is filled with ultrasonic transmission medium. An ultrasonic endoscope is known, and includes the elongated tube. The balloon of an expandable form is used to surround an ultrasonic transducer in the elongated tube, and is filled with ultrasonic transmission medium.

The balloon is formed from rubber or other resilient material. Ends of the balloon are ring portions with a smaller diameter than an outer diameter of a rod device (for example, elongated tube or medical assist device) of a medical instrument in a natural condition. To set the balloon, a diameter of its ends is increased while the balloon is moved to cover the periphery of the rod device. Then a diameter of a rubber ring or ring device is increased and fitted on each of the ends of the balloon. Thus, the ends of the balloon are fixedly positioned on the rod device.

The rubber ring is characteristically required to have great strength in the clamping for the purpose of ensuring air-tightness of the balloon. In contrast, the elongated tube is an instrument of high precision, and cannot be handled roughly. Great force is required for expanding the rubber ring, while the rubber ring must be fitted with fine handling for the precise instrument. Operation of fitting the balloon or the rubber ring around the rod device is very complicated, and requires complicated steps of assembly.

In view of the problem, a guide device of a cylindrical shape is utilized for mounting the rubber ring on the elongated tube. The guide device has an inclined tip at its one end. The rubber ring is entered from the inclined tip, slid on the inclined tip and shifted to a cylindrical surface of the guide device. A diameter of the rubber ring is increased by an outer surface of the cylindrical surface. A tip device of the elongated tube of the endoscope is entered in the guide device. The rubber ring is set in a set position of the guide device, slid from the cylindrical surface, and fitted in a recess of the elongated tube, so as to secure the rubber ring to the elongated tube. The guide device is structurally simple. However, there is a problem in the complicated operation including the three steps which include the expansion of the rubber ring, its movement to the cylindrical surface, and fitting in the recess of the elongated tube.

JP-A 2009-183603 discloses the guide device having four guide rods and a link mechanism. The guide rods contact and spread an inner surface of a ring device (balloon or rubber ring) . The link mechanism shifts the guide rods to increase their diameter, so that the ring device is fitted around the elongated tube or the medical assist device. According to this document, it is possible to increase the diameter of the ring device easily by the special use of the guide device, to facilitate the setting of the ring device on the endoscope or its medical assist device. Also, JP-A 2008-142324 discloses four pins and a link mechanism for increasing the diameter of the pins, in order to set the rubber ring or the balloon on the tip device of the elongated tube. The increase in the diameter of the ring device is facilitated.

U.S. Pat.Pub. Nos. 2011/105840 and 2011/251458 (corresponding to JP-A 2011-517972) disclose the guide device having a cylindrical mechanism and four guide arms, which are supported on the cylindrical mechanism in a pivotally movable manner, in place of the use of the guide rods. The guide arms are pivotally moved to increase the diameter of the rubber ring and the balloon for setting on the tip device of the elongated tube.

However, there is a problem in the guide device disclosed in JP-A 2009-183603 and JP-A 2008-142324 in that an angle of opening and closing a handle must be adjusted for setting the rubber ring, the balloon or the like on the tip device of the elongated tube. Extra operation for advancing the rubber ring from the guide rods is required. Also, according to U. S. Pat. Pub. Nos. 2011/105840 and 2011/251458 (corresponding to JP-A 2011-517972), extra operation for advancing the rubber ring from the guide rods is required. An operator must manipulate with both of his or her hands. The handle in any of those documents must be manipulated finely for opening and closing. The rubber ring must be directly advanced. A problem lies in impossibility in fitting the ring device such as the rubber ring on the elongated tube with a single action, to lower the efficiency. It is necessary for the guide device to have a complicated link mechanism for moving the guide rods, and have an additional pushing mechanism, so that arrangement of elements of the guide device is very complicated.

In accordance with the preamble of claim 1, JP 2009/160093 A discloses an applicator for fitting a resilient ring device around a rod device. This prior art applicator has four arm devices received within oblique holes provided within a disk shaped support means, wherein threaded outer surfaces of the arm devices are engaged with threaded inner surfaces of the support means. The movable sleeve of the prior art applicator can be moved in a circumferential direction so that due to the threaded engagement between the support means and the arm devices, the arm devices are displaced parallel between a closed state of the arm devices and an open state of the arm devices so as to be introduced into the ring device and so as to receive the rod device, respectively.

For advancing the ring device from the arm devices there is needed an extra operation to pull the rod device from the ring device.

### SUMMARY OF THE INVENTION

In view of the foregoing problems, an object of the present invention is to provide an applicator capable of fitting a resilient ring device such as a rubber ring or balloon on an endoscope or other medical instrument in a rapid and exact manner.

In order to achieve the above and other objects and advantages of this invention, an applicator for fitting a resilient ring device around a rod device by radial expansion has the features of claim 1. A movable sleeve receives entry of the rod device. Support means supports the movable sleeve movably in an axial direction. At least three arm devices have a rear end portion, are arranged around the movable sleeve circumferentially, for extending in the axial direction, the rear end portion being secured to the support means in a pivotally movable manner in a radial direction of the movable sleeve. A receiving portion is formed with a front end portion of the arm devices, for receiving an inner edge of the ring device, and moving to open and closed state positions upon pivotal movement of the arm devices, the receiving portion, when in the closed state position, being adapted to setting the ring device thereon, and when in the open state position, being adapted to setting the ring device around the rod device. A shift mechanism shifts the movable sleeve relative to the support means, to move the arm devices pivotally by engaging the movable sleeve with the arm devices, to move the receiving portion from the closed state position to the open state position, and advances the ring device from the receiving portion with an end surface of the movable sleeve.

Each of the at least three arm devices includes an inner surface disposed to extend along the movable sleeve when the receiving portion is in the open state position.

The shift mechanism moves the movable sleeve between first and second positions relative to the support means, and the receiving portion, when the movable sleeve is in the first position, is in the closed state position, and when the movable sleeve is in the second position, is in the open state position.

The shift mechanism includes a grip portion to which the support means is secured. A shift lever is disposed on the grip potion in a pivotally movable manner. An input lever arm is disposed at a first end of the shift lever. An output lever arm is disposed at a second end of the shift lever, for engagement with the movable sleeve.

Furthermore, a cam projection is formed at the rear end portion of the at least three arm devices, for contacting the movable sleeve to prevent pivotal movement. The movable sleeve includes an engaging projection for engagement with the cam projection in the first position, to set the receiving portion in the closed state position.

Furthermore, a first biasing device biases the receiving portion toward the closed state position. The shift mechanism includes a second biasing device for biasing the movable sleeve toward the first position.

In another preferred embodiment, the shift mechanism includes a biasing device for biasing the movable sleeve toward the second position.

In one preferred embodiment, the shift mechanism includes a motor for moving the movable sleeve.

The at least three arm devices include a first arm device, and a second arm device, disposed to extend with a smaller length in the axial direction than the first arm device, and adapted to positioning on the rod device.

In still another preferred embodiment, the at least three arm devices include a first arm device, and a second arm device, disposed to extend with a larger length in the axial direction than the first arm device, for preventing the ring device from incidental loss.

In one preferred embodiment, each of the at least three arm devices further includes a peripheral projection, disposed outside the receiving portion, for preventing the ring device from dropping out of the receiving portion.

Furthermore, a ramped surface is disposed inside the arm devices and on a side of the rear end portion from the receiving portion, inclined relative to the movable sleeve, pushed by the end surface of the movable sleeve when the movable sleeve moves in the axial direction, for facilitating pivotal movement of the arm devices toward the open state position.

Furthermore, at least three receiving grooves are formed in the movable sleeve, the at least three arm devices moving into and out of respectively the receiving grooves when pivotally moved between the open and closed state positions.

The support means includes an arm support sleeve, secured outside the movable sleeve, for supporting the at least three arm devices in a pivotally movable manner.

The rod device is an elongated tube of an endoscope, and the ring device is a rubber ring for securing a balloon to the elongated tube or is a balloon.

Also, an applicator for fitting a resilient ring device around a rod device is provided. A support device has a through channel. A movable sleeve is disposed through the through channel movably in forward and backward directions. At least three arm devices are arranged on a periphery of the movable sleeve in a pivotally movable manner on the movable sleeve between open and closed state positions. A receiving portion is disposed to extend from each of the arm devices in the forward direction, for receiving the ring device outside. A shift mechanism shifts the movable sleeve in the forward direction, and presses the arm devices with an end surface thereof to move pivotally from the closed state position to the open state position, so as to expand the ring device in a radial direction with the receiving portion. When the arm devices are in the open state position, the movable sleeve receives entry of the rod device in the backward direction therein, and the shift mechanism further shifts the movable sleeve in the forward direction, to press the ring device in the forward direction to set the ring device around the rod device.

Consequently, a ring device can be fitted around an endoscope in a rapid and exact manner, because the movable sleeve can cause the arm devices to adjust a diameter of the ring devices suitably.

### BRIEF DESCRIPTION OF THE DRAWINGS

The above objects and advantages of the present invention will become more apparent from the following detailed description when read in connection with the accompanying drawings, in which:
Fig. 1 is a perspective view illustrating an applicator;
Fig. 2 is a vertical section illustrating the applicator;
Fig. 3 is a perspective view illustrating a combination of an elongated tube of an endoscope with rubber rings and a balloon;
Fig. 4 is a front elevation illustrating a set of arm devices in a closed state position with the rubber ring;
Fig. 5 is a vertical section taken on line V-V in Fig. 4;
Fig. 6 is a front elevation illustrating the set of arm devices in an open state position where a diameter of the rubber ring increases;
Fig. 7 is a vertical section taken on line VII-VII in Fig. 6;
Fig. 8 is a perspective view illustrating the applicator with the rubber ring of which the diameter increases;
Fig. 9 is a vertical section illustrating the applicator in which the rubber ring is advanced;
Fig. 10 is a perspective view illustrating the applicator in which the rubber ring is advanced;
Fig. 11 is a vertical section illustrating another preferred applicator having extended receiving portions;
Fig. 12 is a vertical section illustrating another preferred applicator having a fail-safe peripheral projection;
Fig. 13 is an explanatory view illustrating another preferred applicator having a motor;
Fig. 14 is a perspective view illustrating still another preferred applicator in which a torsion coil spring biases a receiving portion toward an open state position;
Fig. 15 is a vertical section illustrating the applicator;
Fig. 16 is a vertical section illustrating arm devices in the open state position;
Fig. 17 is a vertical section illustrating the arm devices in the closed state position;
Fig. 18 is a side elevation illustrating another preferred applicator having a tension coil spring;
Fig. 19 is a perspective view illustrating a further preferred applicator having an annular guide plate for arm devices;
Fig. 20 is a vertical section illustrating arm devices in the open state position;
Fig. 21 is a vertical section illustrating the arm devices in the closed state position;
Fig. 22 is a perspective view illustrating another preferred applicator having a compression coil spring for biasing a movable sleeve.

### DETAILED DESCRIPTION OF THE PREFERRED

### EMBODIMENT(S) OF THE PRESENT INVENTION

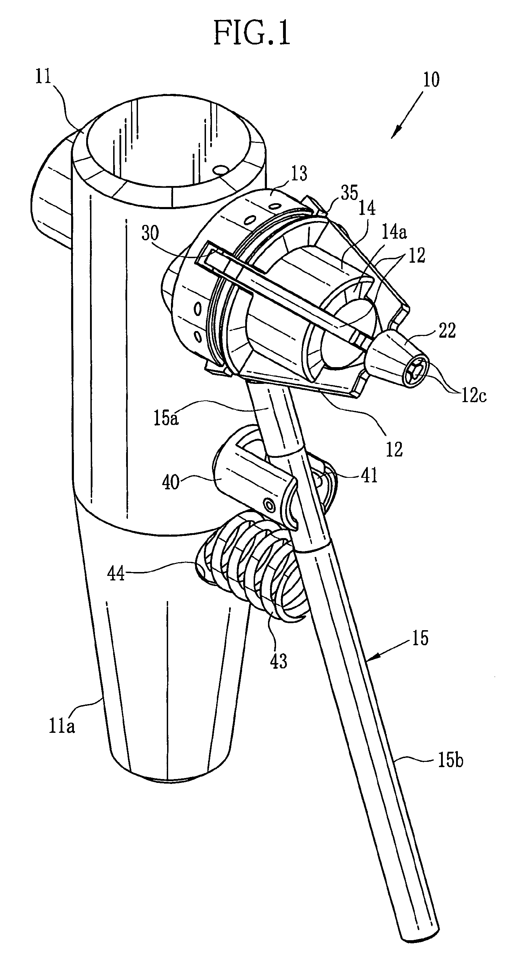

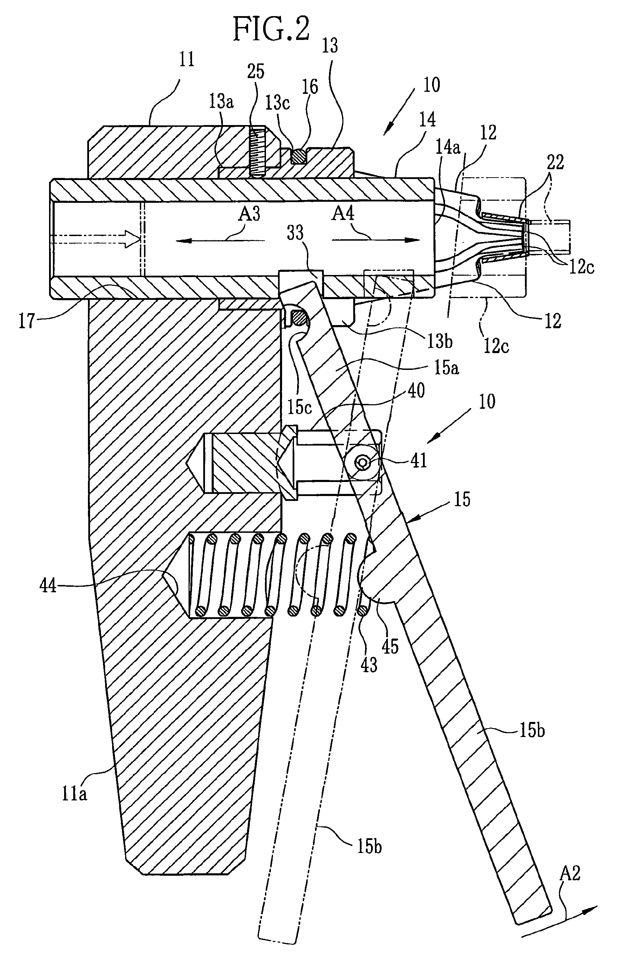

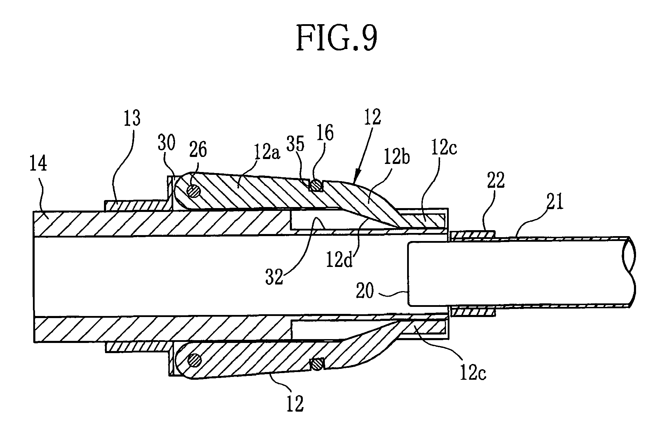

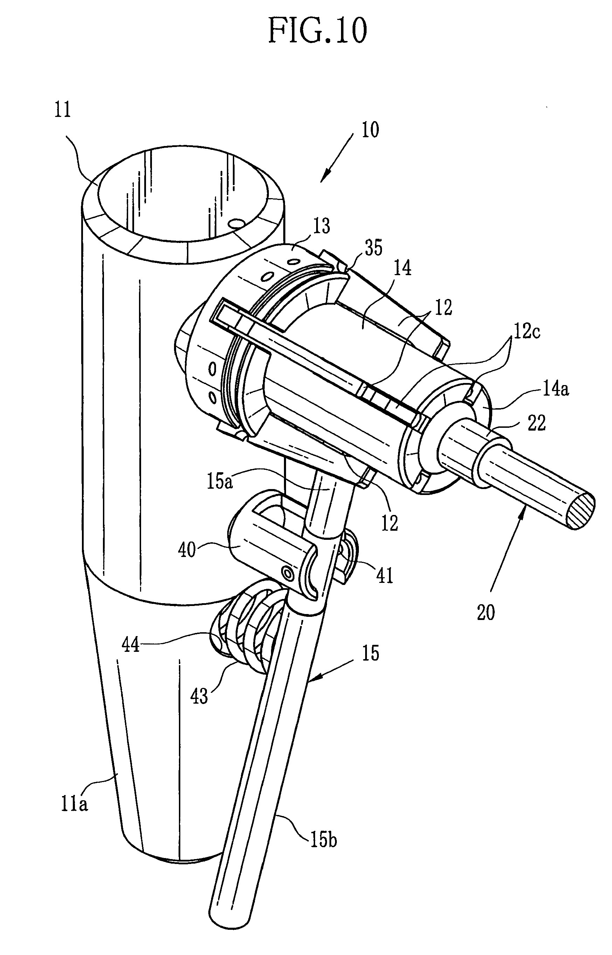

In Figs. 1 and 2, an applicator 10 for fitting a resilient ring device around a rod device includes a support device 11 or support drum for grasping, arm devices 12 for stretch, an arm support sleeve 13 or support shell (boss), a movable sleeve 14, a shift lever 15 as shift mechanism, and a closure ring 16. A through channel 17 is formed through an upper portion of the support device 11 and extends horizontally. A grip 11a is a lower portion of the support device 11 and grasped by a hand for manipulation. The movable sleeve 14 is mounted in the through channel 17 in a slidable manner between a first position (Figs. 1 and 2) and a second position (Figs. 9 and 10).

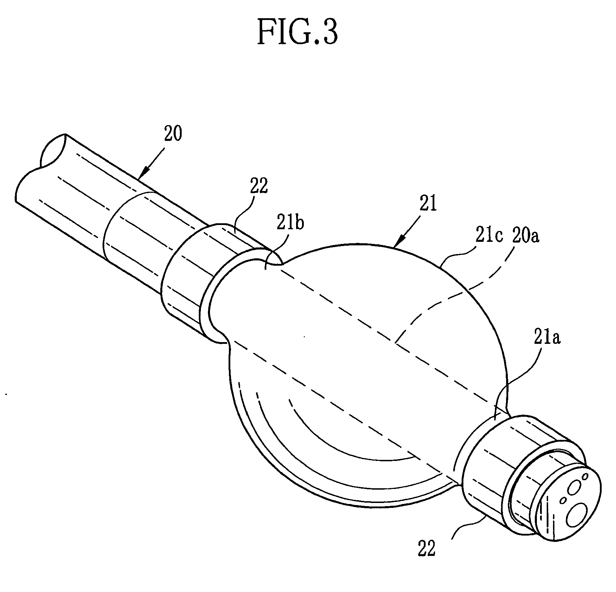

In Fig. 3, an elongated tube 20 of an endoscope includes a tip device 20a. The applicator 10 is used for fitting a balloon device 21, or rubber bands or rubber rings 22 as resilient ring devices on the tip device 20a. The balloon device 21 is formed from resilient material such as silicone rubber, and includes a balloon portion 21c and balloon ends 21a and 21b with a small diameter. The balloon device 21 receives entry of the elongated tube 20 and is placed in a predetermined set position. The rubber rings 22 are fitted on the balloon ends 21a and 21b to keep the balloon device 21 positioned on the elongated tube 20 in a tight manner.

Inner diameters of the rubber rings 22 and the balloon ends 21a and 21b of the balloon device 21 are smaller than an outer diameter of the elongated tube 20 in their natural state without expansion or deformation. The elongated tube 20 cannot be entered in those elements without deformation. In view of this, the applicator 10 of the invention is used to enlarge the diameter of the rubber rings 22 (or the balloon ends 21a and 21b of the balloon device 21) to facilitate entry of the elongated tube 20. The rubber rings 22 (or the balloon ends 21a and 21b) can be fitted on the elongated tube 20 with high precision.

In Fig. 2, the arm support sleeve 13 is secured to the support device 11 and disposed around the movable sleeve 14. The arm support sleeve 13 is cylindrical, and has an annular ridge 13a with a small diameter. While the annular ridge 13a is entered in the through channel 17 of the support device 11, a fastening screw 25 is used to fasten the arm support sleeve 13 in an unremovable manner on the support device 11.

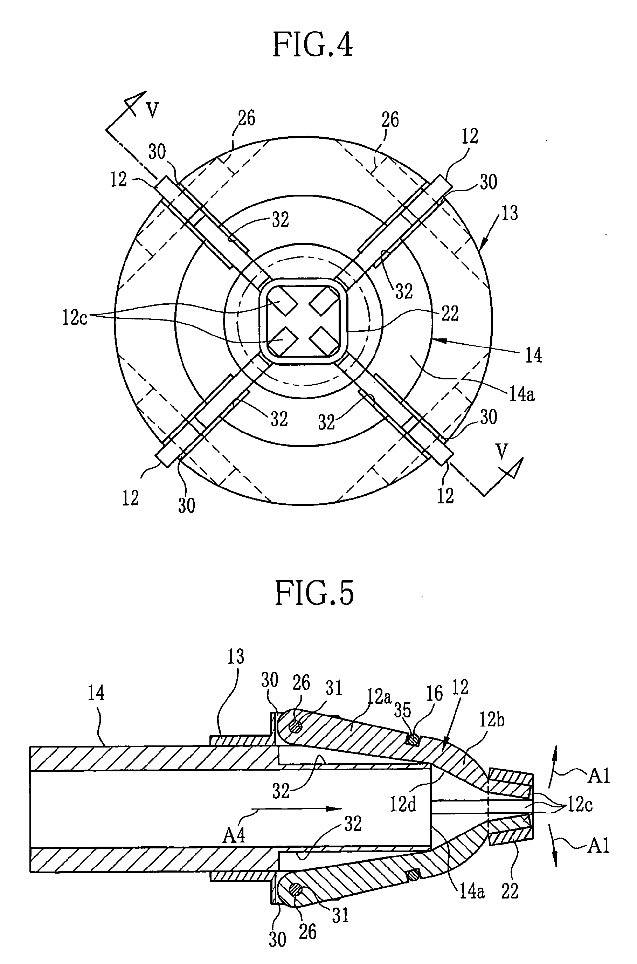

In Fig. 4, four receiving grooves 30 are formed in the arm support sleeve 13 to extend in the radial directions, and arranged in an equiangular manner with 90 degrees. The arm devices 12 are entered in the receiving grooves 30. An arm shaft 26 keeps each of the arm devices 12 rotatable on the arm support sleeve 13.

Receiving grooves 32 are formed in the movable sleeve 14 and aligned with respectively the receiving grooves 30 of the arm support sleeve 13. When the arm devices are received in the receiving grooves 32, their distal ends are in a closed state position with a narrow space. In Fig. 2, a sleeve hole 33 as a transmission device is formed through the movable sleeve 14 and disposed between two of the receiving grooves 32 in the circumferential direction.

In Fig. 5, the arm devices 12 are plates of metal in a strip shape. Each of the arm devices 12 in a crank shape includes a rear end portion 12a, an intermediate portion 12b, and a receiving portion 12c or stretcher head. An axial hole 31 is formed in the rear end portion 12a, and receives entry of the arm shaft 26. At a distal end of the arm devices 12, the receiving portion 12c is disposed for receiving the rubber ring 22 thereabout. A ramped surface 12d of the intermediate portion 12b is engaged with the movable sleeve 14. The arm devices 12 are rotated in radial directions A1 toward an open state position by movement of the movable sleeve 14 in the forward direction A4 (right side) in contact with the ramped surface 12d.

An arcuate cutout 35 is formed in each of the arm devices 12 and near to the axial hole 31. The closure ring 16 or an 0-ring as a biasing device is fitted in the arcuate cutout 35 and disposed outside. An annular groove 13c is formed in the arm support sleeve 13 and receives the closure ring 16. See Fig. 2. The closure ring 16 biases the arm devices 12 internally toward their closed state position. An end surface 14a of the movable sleeve 14 contacts the arm devices 12 and prevents the arm devices 12 from rotating further toward the center. The receiving portions 12c of the arm devices 12 are positioned close to one another (closed state position), so that the rubber ring 22 can be set around the combination of the receiving portions 12c without its radial enlargement. Also, the arm devices 12 are set in the closed state position by the closure ring 16, and thus can be contained compactly in the receiving grooves 30 of the arm support sleeve 13 and the receiving grooves 32 of the movable sleeve 14.

In Fig. 2, a pivoting device 40 is secured at the center of the support device 11. The pivoting device 40 keeps the shift lever 15 rotatable on the grip 11a. The shift lever 15 has an output lever arm 15a disposed higher than a lever shaft 41 of the pivoting device 40, and an input lever arm 15b disposed lower than the lever shaft 41. A cutout 13b is formed in the arm support sleeve 13 through which the output lever arm 15a is entered in the sleeve hole 33 of the movable sleeve 19 . A cutout 15c is formed in the shift lever 15 to avoid its interference with the closure ring 16.

A compression coil spring 43 is disposed between the support device 11 and the input lever arm 15b of the shift lever 15. A receiving hole 44 is formed in the support device 11, and receives a first end of the compression coil spring 43. A coupling projection 45 is formed on the input lever arm 15b. A second end of the compression coil spring 43 is engaged with the coupling projection 45, to bias the shift lever 15 in an outer direction A2 indicated by the arrow. Thus, the input lever arm 15b is normally in its home relative to the support device 11. As the output lever arm 15a is engaged with the sleeve hole 33, the compression coil spring 43 biases the movable sleeve 14 in a backward direction A3 for containment which is opposite to a forward direction A4 for shift. The receiving portions 12c of the arm devices 12 are in the closed state position (first position) of a narrowed state to facilitate the setting of the rubber ring 22 around the receiving portions 12c.

The operation of the applicator 10 for fitting the rubber ring 22 is described in relation to the balloon device 21 for the elongated tube 20. At first, the rubber ring 22 is set around the receiving portions 12c of the arm devices 12 as illustrated in Fig. 1.

Then the grip 11a of the support device 11 is manually grasped to pull the shift lever 15. In Fig. 2, a shift of the shift lever 15 is transmitted through the sleeve hole 33 to the movable sleeve 14. In Fig. 5, the movable sleeve 14 is moved through the through channel 17 in the support device 11 in the forward direction A4 for push. The distal end of the movable sleeve 14 is caused to push the ramped surface 12d of the intermediate portion 12b of the arm devices 12, to shift the arm devices 12 for opening in the radial direction A1. The rubber ring 22 set around the receiving portions 12c is gradually stretched for expansion by the arm devices 12.

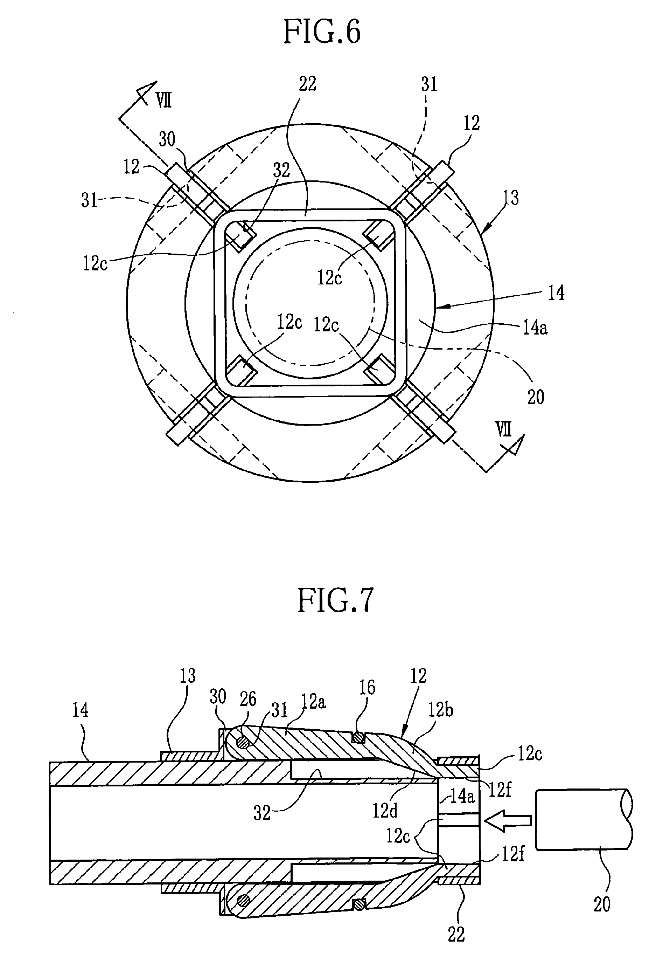

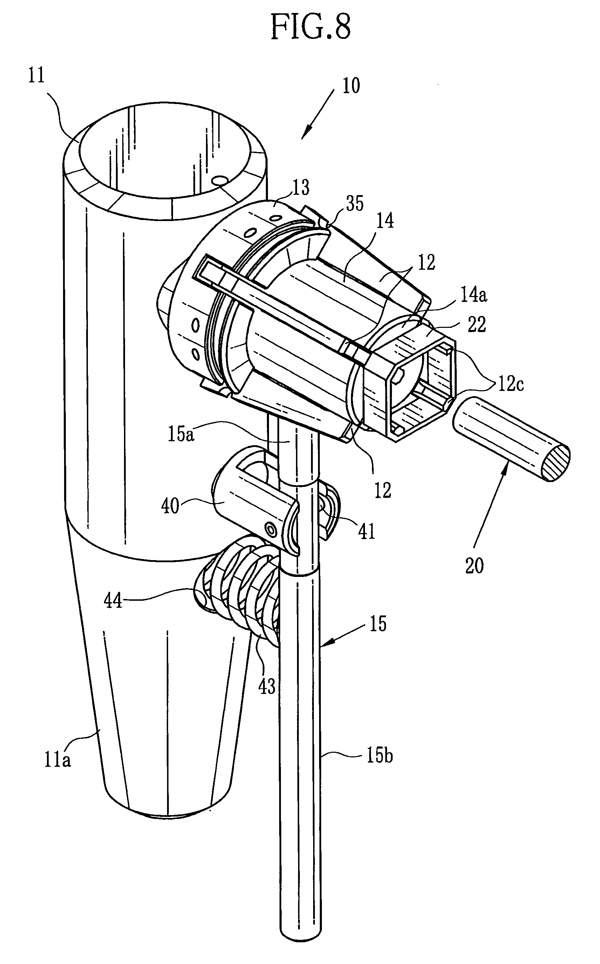

In Fig. 7, the end surface 14a of the movable sleeve 14 moves to pass the ramped surface 12d of the intermediate portion 12b to cause the movable sleeve 14 to contact the intermediate portion 12b. A diameter of the rubber ring 22 is maximized. In Figs. 6 and 8, the diameter of the rubber ring 22 is larger than the outer diameter of the elongated tube 20. Thus, the elongated tube 20 can be entered in the movable sleeve 14. The distal end of the movable sleeve 14 is moved past the intermediate portion 12b of the open state position when advanced by use of the shift lever 15. Then inner surfaces 12f for parallelism in the receiving portion 12c are contacted by the movable sleeve 14.

In this state, the elongated tube 20 is moved back and forth through the movable sleeve 14 to position the rubber ring 22 in the set position. The inner surfaces 12f of the receiving portion 12c are oriented in parallel with the movable sleeve 14 in the state of the maximized diameter (open state position). The inner surfaces 12f, when engaged with the movable sleeve 14, remain in parallel even though the repulsive force of the rubber ring 22 in the return direction of decreasing the diameter is applied to the arm devices 12. Thus, the shift lever 15 can be positioned stably without exertion of the repulsive force in the outer direction of the shift lever 15 for containing the movable sleeve 14.

The shift lever 15 is pulled further to move the movable sleeve 14 in the forward direction A4. The arm devices 12 do not shift further in the radial direction. The rubber ring 22 is slid on the receiving portion 12c by the end surface 14a of the movable sleeve 14. The arm devices 12 are extended in parallel with the movable sleeve 14 by the inner surfaces 12f. Accordingly, the movable sleeve 14 can be pushed out without excessively high force. In Figs. 9 and 10, the end surface 14a of the movable sleeve 14 moves past the distal end of the receiving portion 12c of the arm devices 12. The diameter of the rubber ring 22 is decreased in the set position on the elongated tube 20 because of compression upon the separation from the receiving portion 12c. The rubber ring 22 can be correctly positioned in the set position.

It is possible reliably to enlarge the diameter of the rubber ring 22 and position the rubber ring 22 on the elongated tube 20 only by manipulation of the shift lever 15. The rubber ring 22 can be fitted around the elongated tube 20 easily. It is unnecessary to carry out the sliding step in a separate manner from the stretch step of enlarging the diameter as disclosed in the patent documents JP-A 2009-183603, JP-A 2008-142324 and U.S. Pat.Pub. Nos. 2011/105840 and 2011/251458 (corresponding to JP-A 2011-517972).

In the above embodiment, the arm devices 12 are the four disposed equiangularly from one another with reference to the arm support sleeve 13. However, the number of the arm devices 12 can be at least three. A diameter of the inner surface of the rubber ring 22 can be set larger than the outer diameter of the elongated tube 20.

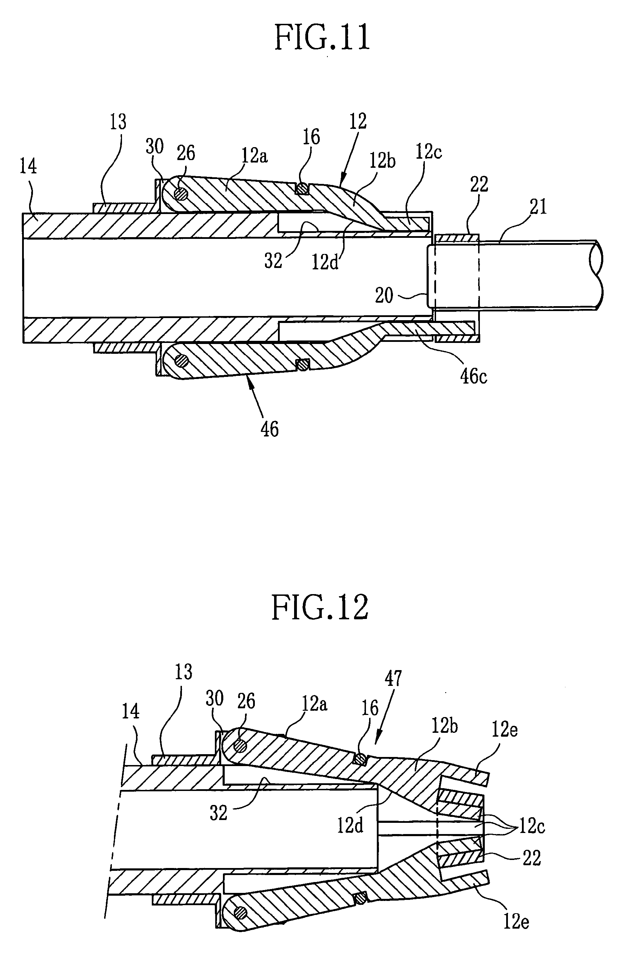

A second preferred embodiment is described now. In Fig. 11, the receiving portion 12c of only one arm device 12 is equal to that of the first embodiment. Three remaining arm devices 46 for stretch have a receiving portion with a length substantially two times as large as the receiving portion 12c of the arm device 12 of the first embodiment. A movement range of the movable sleeve 14 is enlarged for the extension of the arm devices 46 over a movement range of the same according to the first embodiment. Elements similar to those of the first embodiment are designated with identical reference numerals. In the second embodiment, the rubber ring 22 is removed from the receiving portion 12c in the course of movement of the movable sleeve 14, to contact a portion of a set position in the elongated tube 20. It is possible correctly to recognize the set position from a position of the rubber ring 22 in contact with the elongated tube 20, and to adjust the set position more finely. Accordingly, precision in positioning the rubber ring 22 can be considerably high. Each of the arm devices 46 has a receiving portion 46c (stretcher head) . When the movable sleeve 14 is moved further, the rubber ring 22 is removed from the receiving portion 46c, to fit the rubber ring 22 on the elongated tube 20 in its correct set position.

The three remaining arm devices 46 are longer than the particular arm device 12. Thus, the possibility of drop of the rubber ring 22 from the receiving portions 46c can be decreased. Loss of the rubber ring 22 from the receiving portions 46c can be prevented during the stretch with an increasing diameter of the rubber ring 22. Note that at least one of the four arm devices 12 and 46 can have a greater length for the purpose of prevention of the loss. For such a structure, the movable sleeve 14 is moved until the rubber ring 22 slides from the arm devices 46 and compresses around the elongated tube 20.

A third preferred embodiment is described now by referring to Fig. 12. A set of arm devices 47 for stretch is provided in a manner different from the arm devices 12. A fail-safe peripheral projection 12e of each of the arm devices 47 is disposed outside the receiving portion 12c for preventing the rubber ring 22 from incidental loss. Should the rubber ring 22 be offset from the receiving portion 12c, the peripheral projection 12e can push the rubber ring 22 and keep the same in contact with the receiving portion 12c. So the rubber ring 22 can be prevented from dropping incidentally. Only one of the arm devices 47 may have the peripheral projection 12e. However, it is preferable for all of the arm devices 47 to have the peripheral projection 12e for reliability in the prevention of the incidental loss.

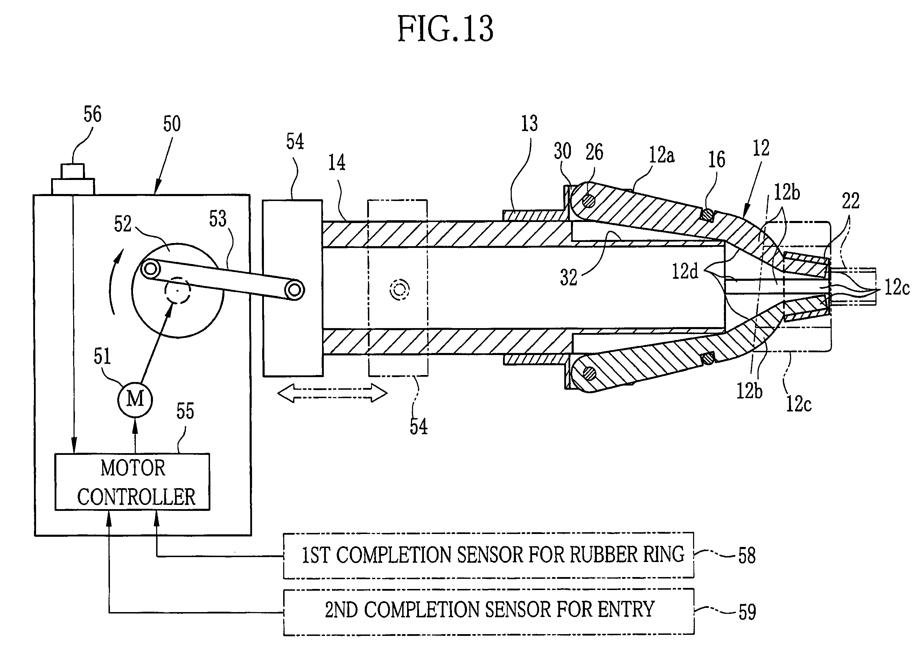

In Fig. 13, a shift mechanism 50 of a fourth preferred embodiment is illustrated in place of the shift lever 15 for shifting the movable sleeve 14. The shift mechanism 50 includes a motor 51, a cam 52, a link shaft 53, a holder ring 54, a motor controller 55 and an actuating button 56 for shift. The actuating button 56 is depressed after setting of the rubber ring 22 around the receiving portions 12c in the closed state position. The motor controller 55 causes the motor 51 to rotate at a predetermined amount. The motor 51 rotates to move the movable sleeve 14 in the forward direction. The arm devices 12 are pivotally moved to the open state position, so that the diameter of the rubber ring 22 is maximized by stretch. In the open state, the elongated tube 20 is entered in the movable sleeve 14 in the backward direction, to position the rubber ring 22 in the set position of the elongated tube 20. Then the actuating button 56 is depressed to rotate the motor 51 at a predetermined amount. The movable sleeve 14 is moved further to push the rubber ring 22 from the receiving portions 12c by use of the end surface 14a of the movable sleeve 14. The rubber ring 22 is readily fitted on the elongated tube 20 in its predetermined position in a semi-automatic manner.

Furthermore, it is possible in the shift mechanism 50 to use a rack/pinion structure, worm gear structure and the like for transmission, in place of the cam 52 and its relevant elements.

Furthermore, it is possible instead of the actuating button 56 to dispose a first completion sensor 58 for a rubber ring and/or a second completion sensor 59 for entry. The first completion sensor 58 detects completion of setting of the rubber ring 22 around the receiving portions 12c. The second completion sensor 59 detects entry of the elongated tube 20 in the movable sleeve 14 at a predetermined length. Signals from the first completion sensor 58 and the second completion sensor 59 are input instead of the control signal from the actuating button 56, so that the rubber ring 22 can be automatically fitted on the elongated tube 20 in the set position.

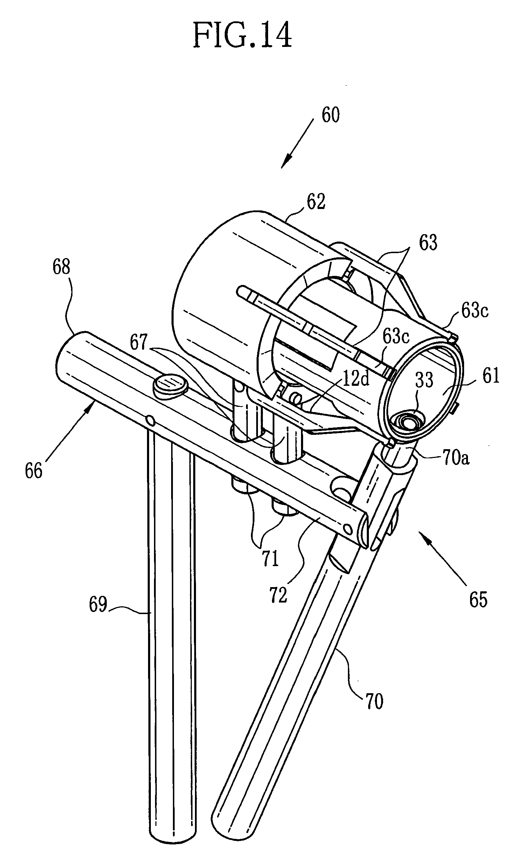

In Fig. 14, a fifth preferred embodiment is illustrated. In the first embodiment, the shift lever 15 is biased by the compression coil spring 43 for setting the receiving portion 12c of the arm devices 12 in the first position (closed) with a reduced diameter. The receiving portion 12c with the tip of the small width is disposed normally to protrude from the distal end of the movable sleeve 14. Shock may occur on the applicator 10, as the applicator 10 is manipulated directly by a hand of the doctor or operator, and may be dropped or struck by other objects. The arm devices 12 have a long form with the receiving portion 12c. Deformation or breakage of the arm devices 12 may occur upon receiving shock as a problem of durability.

In the first embodiment, the shift mechanism is constituted by the support device 11, the shift lever 15 and the closure ring 16. Significant abrasion of the closure ring 16 may occur as 0-ring of rubber or elastomer, because its portion contacting the outside of the arm devices 12 is repeatedly rubbed or expanded. Resistance to heat and durability may not be maintained due to the rubber for the closure ring 16, typically upon receiving thermal load of autoclave sterilization for medical use.

In Fig. 14, an applicator 60 of the fifth embodiment includes a movable sleeve 61 and arm devices 63 for stretch. A receiving portion 63c of the arm devices 63 is in the second position (open) in a normal state, and is so supported on the movable sleeve 61 that shock to the receiving portion 63c can be reduced by the support of the movable sleeve 61.

The applicator 60 is constituted by the movable sleeve 61, an arm support sleeve 62 or support shell (boss), the arm devices 63, the receiving portion 63c and a shift mechanism 65. The elements of the first embodiment are repeated in the applicator 60 but with a difference of the shift mechanism 65.

A support device 66 for grasping is provided in the shift mechanism 65, and attached to the arm support sleeve 62. The support device 66 includes two support posts 67, a support drum 68 or support board, a vertically extending grip 69, and a shift lever 70.

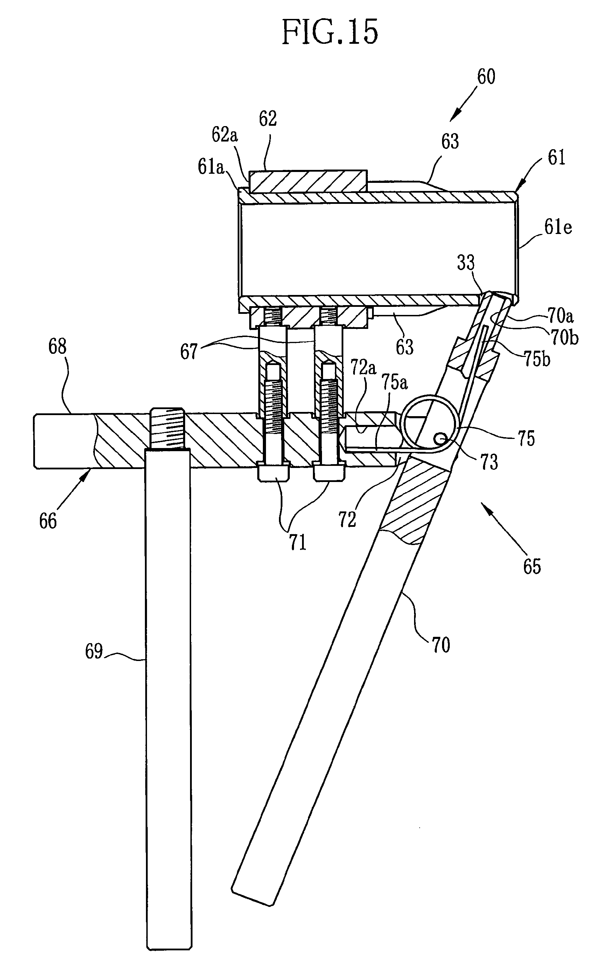

In Fig. 15, screws 71 are disposed for attaching lower ends of the support posts 67 to the support drum 68. Also, the support posts 67 have threads formed at their upper tips, and are attached to a lower wall of the arm support sleeve 62 with the threads. To a rear end portion of the support drum 68, an upper end portion of the grip 69 is attached by engagement of threads. A pivoting device 72 or lever support in a fork shape is formed with a front end of the support drum 68. A lever shaft 73 in the pivoting device 72 keeps the shift lever 70 rotatable. A combination of the support posts 67, the support drum 68 and the grip 69 is manually handled by a user or operator.

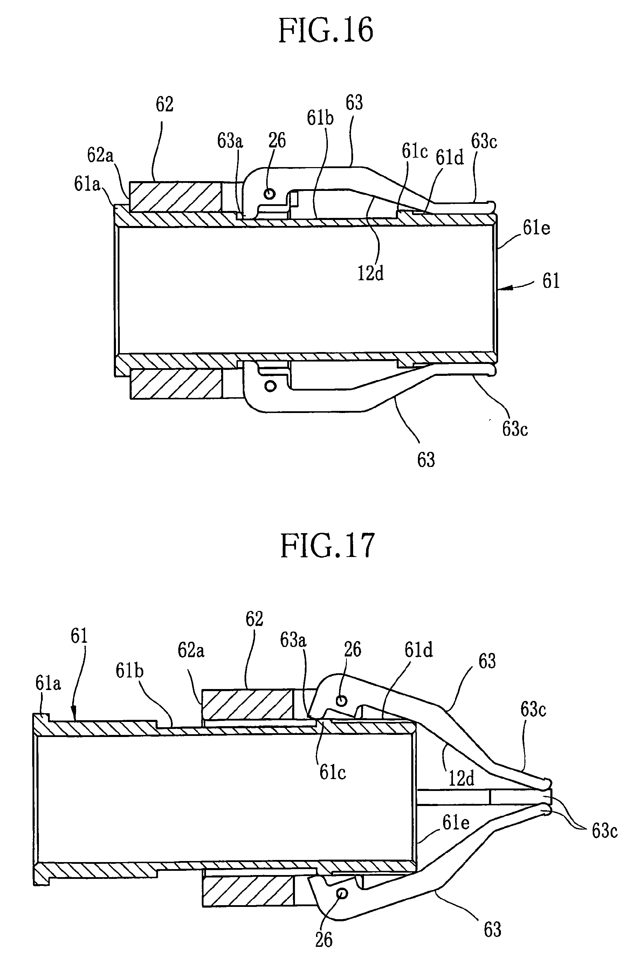

In Fig. 16, a radial flange 61a or stopping flange is formed at a rear end of the movable sleeve 61. An end surface 62a of the arm support sleeve 62 is contacted by the radial flange 61a, and prevents the movable sleeve 61 from moving further through the arm support sleeve 62. The movable sleeve 61 is in the second position when the radial flange 61a is stopped by the end surface 62a. The arm devices 63 extend along the outer surface of the movable sleeve 61 in the second position. The receiving portion 63c is positioned on the outer surface of the movable sleeve 61.

In Fig. 15, the sleeve hole 33 is formed through the wall at the front end of the movable sleeve 61. An output lever arm 70a of the shift lever 70 is entered in the sleeve hole 33. The output lever arm 70a is constituted by a root portion and an end sleeve portion formed on the root portion with a smaller width.

The shift mechanism 65 includes a torsion coil spring 75 for operating the pivoting device 72 and the shift lever 70. The torsion coil spring 75 has a first spring end 75a and a second spring end 75b. A receiving hole 72a is formed in the pivoting device 72, and receives the first spring end 75a for retention. A receiving hole 70b is formed in the output lever arm 70a of the shift lever 70, and receives the second spring end 75b for retention. The torsion coil spring 75 operates for biasing to direct the spring ends 75a and 75b away from one another. The movable sleeve 61 shifts to the right relative to the arm support sleeve 62, and comes to the second position depicted in Figs. 15 and 16. The arm devices 63 are positioned outside the movable sleeve 61. The receiving portion 63c is in the open state position.

In Fig. 16, each of the arm devices 63 includes a cam projection 63a. The movable sleeve 61 includes a recessed surface 61b for sliding, an engaging projection 61c with a cam surface, and receiving grooves 61d. The cam projection 63a is disposed close to the arm shaft of the arm devices 63, and contacts the recessed surface 61b of the movable sleeve 61 for keeping the receiving portion 63c in contact with the outer surface of the movable sleeve 61.

The recessed surface 61b is formed in the movable sleeve 61 and in grooves extending in the axial direction. When the movable sleeve 61 shifts through the arm support sleeve 62, the cam projection 63a contacts the recessed surface 61b, so that the arm devices 63 are kept in the open state position to extend in the axial direction without pivotal movement.

The engaging projection 61c is formed on the movable sleeve 61 and disposed next to the recessed surface 61b in the forward direction. When the movable sleeve 61 is moved to the first position of Fig. 17, the cam projection 63a becomes engaged with and pushed up by the engaging projection 61c to rotate the arm devices 63 toward the closed state position of the receiving portion 63c.

Therefore, it is unnecessary to provide the closure ring 16 of the first embodiment for internally biasing the arm devices 63 to prevent their opening movement. The number of the parts can be reduced. Durability can be kept high without drop due to abrasion or friction, because of the lack of the closure ring 16. It is possible to maintain resistance to heat and durability even upon receiving thermal load of autoclave sterilization.

The initial state of the movable sleeve 61 with the bias of the spring is opposite to that according to the first embodiment. While the shift lever 70 is free before shift, the movable sleeve 61 is set in the second position by the torsion coil spring 75. The arm devices 63 with the receiving portion 63c are initially set along the outer surface of the movable sleeve 61 by contact of the cam projection 63a with the recessed surface 61b. Consequently, deformation or breakage of the applicator 60 can be prevented even upon accidentally receiving shock or contact of other objects. Durability of the applicator 60 can be high.

To set the rubber ring 22 around the receiving portion 63c, the shift lever 70 is shifted to slide the movable sleeve 61 in the arm support sleeve 62 to the first position. Thus, the arm devices 63 protrude from the front end of the movable sleeve 61. The receiving portions 63c are set in the closed state position by engagement of the cam projection 63a with the engaging projection 61c. The shift lever 70 is shifted back after setting the rubber ring 22 around the receiving portions 63c, to contain a rear portion of the movable sleeve 61 in the arm support sleeve 62. The arm devices 63 are rotated toward the open state position by their engagement with the movable sleeve 61. Thus, the receiving portions 63c come to the open state position. In the open state position, the rubber ring 22 is moved to a set position on the elongated tube 20 by entry of the tip device 20a in the movable sleeve 61. Then an end surface 61e of the movable sleeve 61 is utilized by shifting back the shift lever 70, so that the rubber ring 22 is advanced from the receiving portion 63c and can be fitted around the elongated tube 20.

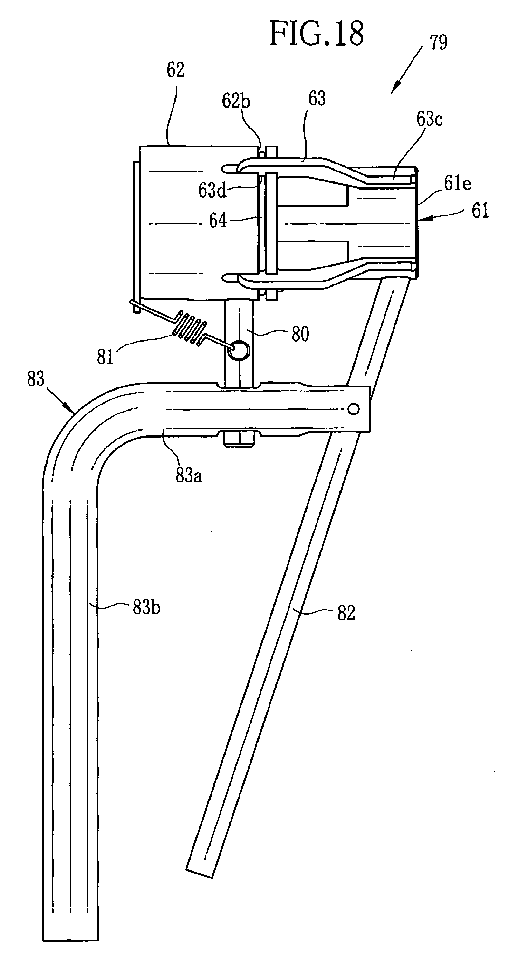

A structure for biasing can be a spring other than the torsion coil spring 75 which biases the movable sleeve 61 toward the second position in the arm support sleeve 62 in the fifth embodiment of Fig. 15. In Fig. 18, an applicator 79 of a sixth preferred embodiment is illustrated, and includes a support post 80 and a tension coil spring 81, which is disposed between the rear end of the movable sleeve 61 and the support post 80, and biases the movable sleeve 61 to the second position. This is effective in simplifying the construction of a shift lever 82 without use of the torsion coil spring 75. A support device 83 for grasping of the applicator 79 includes a support drum 83a or support board, and a vertically extending grip 83b formed together with the support drum 83a in an L shape. The support device 83 can have a simple structure. A receiving channel 62b is formed in an outer surface of the arm support sleeve 62 in an arcuate shape. A clamping spring 64 of metal in a C shape is positioned within the receiving channel 62b. A hole 63d (recess or notch) is formed in the arm devices 63. The clamping spring 64 is disposed to extend through the hole 63d, and biases the arm devices 63 to set the receiving portion 63c in the closed state position.

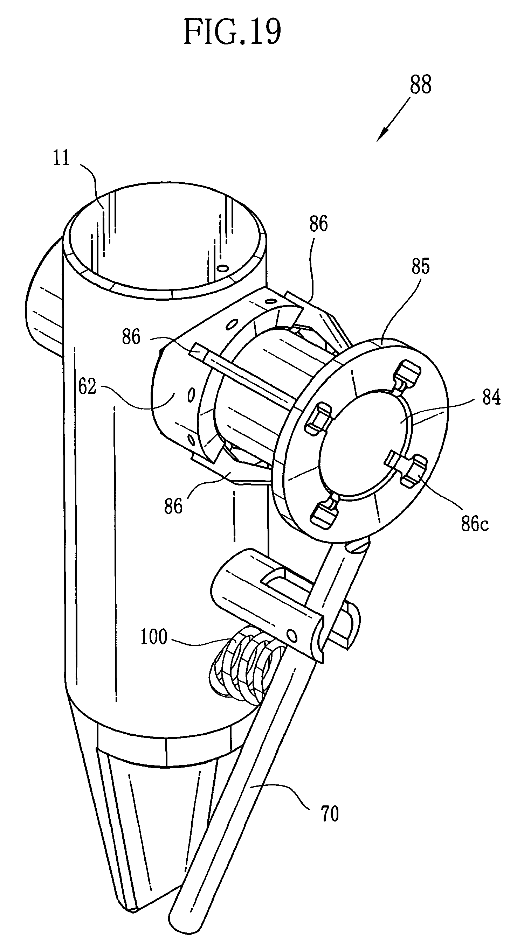

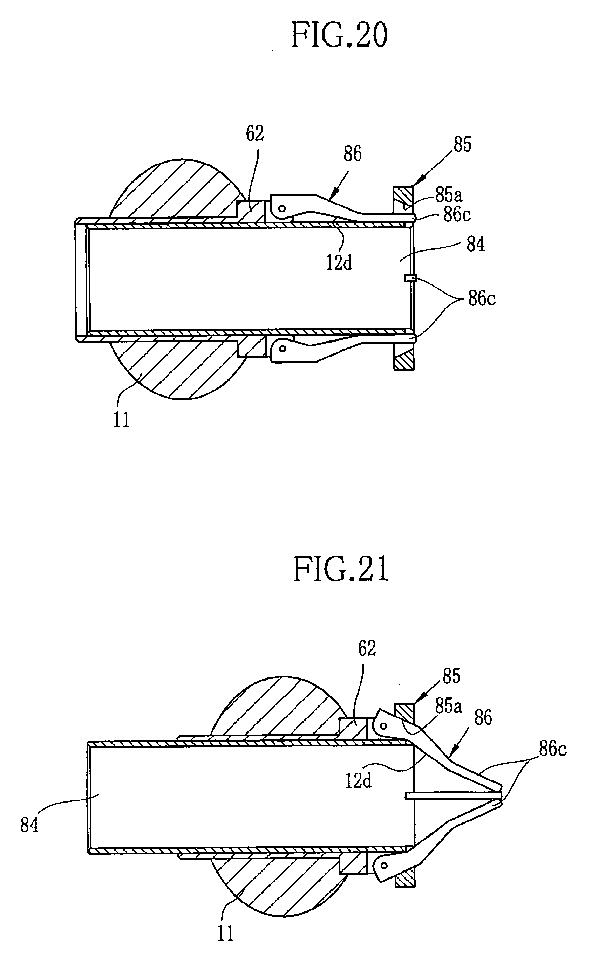

In Figs. 19-21, an applicator 88 of a seventh preferred embodiment is illustrated. The applicator 88 includes a movable sleeve 84, an annular guide plate 85, and arm devices 86 for stretch. In contrast with the fifth embodiment where the arm devices 63 are moved by engagement of the cam projection 63a with the recessed surface 61b and the engaging projection 61c, the annular guide plate 85 for the arms is fixedly positioned on a front end of the movable sleeve 84. Each of the arm devices 86 has a receiving portion 86c. In Fig. 21, the receiving portion 86c is moved to the closed state position by an inner tapered surface 85a of the annular guide plate 85 or a projection and the outside of the arm devices 86. When the arm devices 86 are in the second position (in Figs. 19 and 20) and set on the outside of the movable sleeve 84, the arm devices 86 with the receiving portion 86c are covered and protected by the annular guide plate 85. Thus, flexure resistance of the arm devices 86 against shock upon dropping or contact can be higher. Note that the movable sleeve 84 can be preferably biased to the second position by use of a tension coil spring 100 or other elements for biasing.

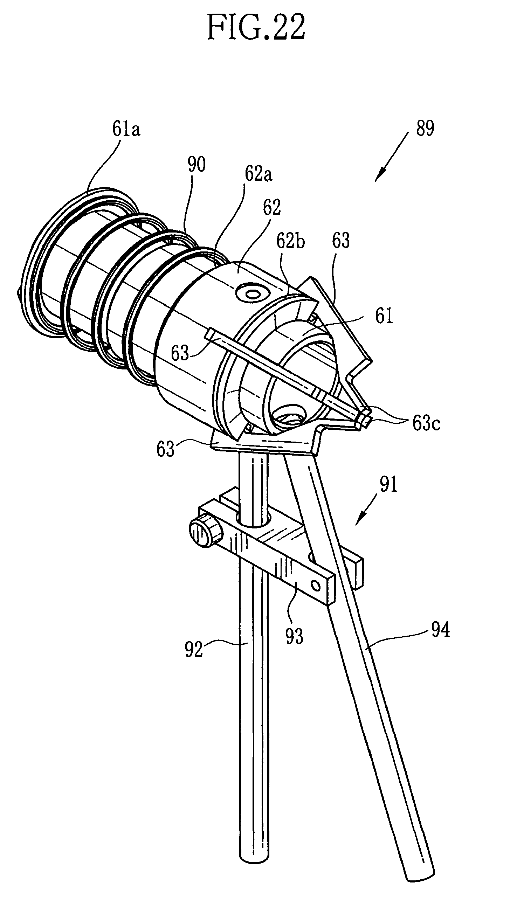

In Fig. 22, an applicator 89 of an eighth preferred embodiment is illustrated. The fifth embodiment is repeated but with a compression coil spring 90 between the radial flange 61a of the movable sleeve 61 and the end surface 62a of the arm support sleeve 62. The movable sleeve 61 is biased by the compression coil spring 90 toward the first position. Resistance to shock according to the applicator 89 is not so high as that of the fifth embodiment, as the receiving portion 63c appears externally from the end of the movable sleeve 61 when in the first position or closed state position as an initial state. However, it is possible to set the rubber ring 22 on the elongated tube 20 of Fig. 3 with manual operability similar to that of the first embodiment. A shift mechanism 91 includes a support post 92, a pivoting device 93 or lever support, and a shift lever 94.

Furthermore, various structures other than the closure ring 16 can be combined with the movable sleeve 14 and the arm devices 12 of the first embodiment, such as the cam projection 63a, the recessed surface 61b and the engaging projection 61c of the fifth embodiment, the annular guide plate 85 of the eighth embodiment, and the like. It is possible to maintain resistance to heat and durability even upon the heat sterilization. Also, the clamping spring 64 of metal in Fig. 18 can be used in place of the closure ring 16 effectively for maintaining resistance to heat and durability. To this end, the hole 63d is formed in the arm devices 12. The clamping spring 64 is positioned in the hole 63d and biases the arm devices 12 to the closed state position. Furthermore, a torsion coil spring (not shown) can be associated with each of the arm devices for biasing toward the closed state position.

In the above embodiments, the recessed surface 61b is formed for guiding the cam projection 63a. However, the recessed surface 61b may not be formed. The cam projection 63a may directly contact the outer surface of the movable sleeve 61. Also, it is possible in the fifth to eighth embodiments to use the shift mechanism 50 of the fourth embodiment in Fig. 13, for driving with the motor 51.

In the above embodiments, the ring devices are the rubber rings 22. However, it is possible in the invention to fit the balloon device 21 to an endoscope by use of the applicator 10. In the above embodiments, the rod device is the elongated tube 20 of the endoscope. However, a rod device for use with the applicator 10 can be a medical assist device in a rod shape, such as an overtube device and sliding tube device, a diagnosis device such as an ultrasonic probe, a treatment device, a catheter, and the like. In the above embodiments, the balloon device 21 and the rubber rings 22 have the ring shapes. However, a ring device to be fitted according to the invention can be a bag-shaped balloon and the like which can have at least partially annular shape with resilient property.

Although the rubber ring 22 is fitted around the elongated tube 20 in the above embodiments, a ring device to be fitted by the applicator 10 may be an 0-ring or other resilient ring for various purposes.

Note that the arm support sleeve 13 or 62 can be formed together with the support device 11, 66 or 83 as support means.

In the above embodiment, the ramped surface 12d has a different inclination from an inner arm surface of the rear end portion 12a disposed in the backward direction. However, the ramped surface 12d can be inclined as a single flat surface extending from the rear end portion 12a to the intermediate portion 12b.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. An applicator for fitting a resilient ring device (21, 22) around a rod device (20, 20a) by radial expansion, comprising:
a movable sleeve (14, 61, 84) for receiving entry of said rod device;
support means (11, 13, 62, 69, 83, 92) for supporting said movable sleeve movably;
at least three arm devices (12, 46, 47, 63, 86), having a rear end portion (12a), arranged around said movable sleeve circumferentially, for extending in said axial direction, said rear end portion being secured to said support means in a radial direction (A1) of said movable sleeve;
a receiving portion (12c, 46c, 63c, 86c), formed with a front end portion of said arm devices, for receiving an inner edge of said ring device, and moving to open and closed state positions upon pivotal movement of said arm devices, said receiving portion, when in said closed state position, being adapted to setting said ring device thereon, and when in said open state position, being adapted to set said ring device around said rod device;
a shift mechanism (15, 50, 70, 82, 94) for shifting said movable sleeve relative to said support means, to move said arm devices by engaging said movable sleeve with said arm devices, to move said receiving portion from said closed state position to said open state position,
**characterized in that** the support means supports the movable sleeve movably in an axial direction (A3, A4); that the rear end portion is secured to the support means in a pivotally movable manner, that the shift mechanism is adapted to shift the movable sleeve so as to move the arm devices pivotally and to advance said ring device from said receiving portion with an end surface of said movable sleeve (14, 61, 84).

2. An applicator as defined in claim 1, **characterized in that** each of said at least three arm devices (12, 46, 47, 63, 86) includes an inner surface disposed to extend along said movable sleeve when said receiving portion is in said open state position.

3. An applicator as defined in claim 1 or 2, **characterized in that** said shift mechanism (15, 50, 70, 82, 94) moves said movable sleeve (14, 61, 84) between first and second positions relative to said support means (11, 13, 62, 83, 92), and said receiving portion (12c, 46c, 63c, 86c), when said movable sleeve is in said first position is in said closed state position, and when said movable sleeve (14, 61, 84) is in said second position, is in said open state position.

4. An applicator as defined in any one of claims 1-3, **characterized in that** said shift mechanism includes:
a grip portion (11a, 69, 83, 92) to which said support means is secured;
a shift lever (15, 70, 82, 94) disposed on said grip potion (11a) in a pivotally movable manner;
an input lever arm (15b) disposed at a first end of said shift lever (15);
an output lever arm (15a), disposed at a second end of said shift lever (15), for engagement with said movable sleeve (14, 61, 84).

5. An applicator as defined in any one of claims 1-4, **characterized in** further comprising a cam projection (63a), formed at said rear end portion of said at least three arm devices (63), for contacting said movable sleeve (61) to prevent pivotal movement;
wherein said movable sleeve (61) includes an engaging projection (61c) for engagement with said cam projection (63a) in said first position, to set said receiving portion (63c) in said closed state position.

6. An applicator as defined in any one of claims 3-5, **characterized in** further comprising a first biasing device (16, 81) for biasing said receiving portion (63c) toward said closed state position;
wherein said shift mechanism (70) includes a second biasing device (43) for biasing said movable sleeve (61) toward said first position.

7. An applicator as defined in any one of claims 3-5, **characterized in that** said shift mechanism includes a biasing device (75) for biasing said movable sleeve (61) toward said second position.

8. An applicator as defined in any one of claims 1-5, **characterized in that** said shift mechanism includes a motor (51) for moving said movable sleeve (14).

9. An applicator as defined in any one of claims 1-8, **characterized in that** said at least three arm devices include a first arm device (12), and a second arm device (46), disposed to extend with a smaller length in said axial direction than said first arm device (12), and adapted to positioning on said rod device (20, 20a).

10. An applicator as defined in any one of claims 1-8, **characterized in that** said at least three arm devices include a first arm device (12), and a second arm device (46), disposed to extend with a larger length in said axial direction than said first arm device (12), for preventing said ring device (21, 22) from incidental loss.

11. An applicator as defined in any one of claims 1-10, **characterized in that** each of said at least three arm devices (12) further includes a peripheral projection (12c), disposed outside said receiving portion (12c), for preventing said ring device (21, 22) from dropping out of said receiving portion (12c).

12. An applicator as defined in any one of claims 1-11, **characterized in** further comprising a ramped surface (12d), disposed inside said arm devices (12) and on a side of said rear end portion from said receiving portion (12c), inclined relative to said movable sleeve (14), pushed by said end surface (14a) of said movable sleeve (14) when said movable sleeve (14) moves in said axial direction, for facilitating pivotal movement of said arm devices (12) toward said open state position.

13. An applicator as defined in any one of claims 1-12, **characterized in** further comprising at least three receiving grooves (30) formed in said movable sleeve (14), said at least three arm devices (12) moving into and out of respectively said receiving grooves (30) when pivotally moved between said open and closed state positions.

14. An applicator as defined in any one of claims 1-13, **characterized in that** said support means includes an arm support sleeve (13), secured outside said movable sleeve (14), for supporting said at least three arm devices (12) in a pivotally movable manner.

## Patentansprüche

1. Applikator zum Aufsetzen einer elastischen Ringanordnung (21, 22) auf eine Stabstruktur (20, 20a) durch radiales Aufweiten, umfassend:
eine bewegliche Hülse (14, 61, 84) zum Aufnehmen des Eintritts der Stabstruktur;
eine Trägereinrichtung (11, 13, 62, 69, 83, 92) zum beweglichen Halten der beweglichen Hülse;
mindestens drei Arme (12, 46, 47, 63, 86) mit einem hinteren Endabschnitt (12a), angeordnet um den Umfang der beweglichen Hülse, um sich in der axialen Richtung zu erstrecken, wobei der hintere Endabschnitt an der Trägereinrichtung in radialer Richtung (A1) der beweglichen Hülse befestigt ist;
einen Aufnahmeabschnitt (12c, 46c, 63c, 86c), ausgebildet mit einem vorderen Endabschnitt der Arme, um eine Innenkante der Ringanordnung aufzunehmen und sich bei einer Schwenkbewegung der Arme in eine Offen- und in eine Schließstellung zu bewegen, wobei der Aufnahmeabschnitt in der Schließstellung dazu ausgebildet ist, auf sich die Ringanordnung aufzunehmen, und in der Offenstellung dazu ausgebildet ist, die Ringanordnung um die Stabstruktur herum einzustellen;
einen Verschiebemechanismus (15, 50, 70, 82, 94) zum Verschieben der beweglichen Hülse relativ zu der Trägereinrichtung, um die Arme zu bewegen durch In-Eingriff-Bringen der beweglichen Hülse mit den Armen, um den Aufnahmeabschnitt aus der Schließstellung in die Offenstellung zu bewegen,
**dadurch gekennzeichnet, dass** die Trägereinrichtung die bewegliche Hülse in axialer Richtung (A3, A4) beweglich lagert, dass der hintere Endabschnitt an der Trägereinrichtung schwenkbar befestigt ist, dass der Verschiebemechanismus dazu ausgebildet ist, die bewegliche Hülse so zu verschieben, dass die Arme verschwenkt werden und die Ringanordnung aus dem Aufnahmeabschnitt mit einer Stirnfläche der beweglichen Hülse (14, 61, 84) vorgerückt wird.

2. Applikator nach Anspruch 1, **dadurch gekennzeichnet, dass** jeder der mindestens drei Arme (12, 46, 47, 63, 86) eine Innenfläche enthält, die so angeordnet ist, dass sie sich entlang der beweglichen Hülse erstreckt, wenn der Aufnahmeabschnitt sich in der Offenstellung befindet.

3. Applikator nach Anspruch 1 oder 2, **dadurch gekennzeichnet, dass** der Verschiebemechanismus (15, 50, 70, 82, 94) die bewegliche Hülse (14, 61, 84) zwischen einer ersten und einer zweiten Stellung relativ zu der Trägereinrichtung (11, 13, 62, 83, 92) bewegt, und der Aufnahmeabschnitt (12c, 46c, 63c, 86c), wenn die bewegliche Hülse sich in der ersten Stellung befindet, die Schließstellung einnimmt, und wenn die bewegliche Hülse (14, 61, 84) sich in der zweiten Stellung befindet, die Offenstellung einnimmt.

4. Applikator nach einem der Ansprüche 1 bis 3, **dadurch gekennzeichnet, dass** der Verschiebemechanismus enthält:
ein Griffteil (11 a, 69, 83, 92), an welchem die Trägereinrichtung angebracht ist;
einen Verschiebehebel (15, 70, 82, 94), der an dem Griffteil (11a) verschwenkbar angeordnet ist;
einen Eingangshebelarm (15b), der an einem ersten Ende des Verschiebehebels (15) angeordnet ist;
einen Ausgangshebelarm (15a), der an einem zweiten Ende des Verschiebehebels (15) angeordnet ist, um mit der beweglichen Hülse (14, 61, 84) in Eingriff zu treten.

5. Applikator nach einem der Ansprüche 1 bis 4, weiterhin **gekennzeichnet durch** einen Steuerkurvenvorsprung (63a), der an dem hinteren Endabschnitt der mindestens drei Arme (63) ausgebildet ist, um die bewegliche Hülse (61) zum Unterbinden einer Schwenkbewegung zu berühren;
wobei die bewegliche Hülse (61) einen Eingriffsvorsprung (61 c) für den Eingriff mit dem Steuerkurvenvorsprung (63a) in der ersten Stellung aufweist, um den Aufnahmeabschnitt (63c) in die Schließstellung zu bringen.

6. Applikator nach einem der Ansprüche 3 bis 5, **dadurch gekennzeichnet, dass** er weiterhin eine erste Vorspanneinrichtung aufweist zum Vorspannen des Aufnahmeabschnitts (63c) in Richtung der Schließstellung;
wobei der Verschiebemechanismus (70) eine zweite Vorspanneinrichtung (43) aufweist zum Vorspannen der beweglichen Hülse (61) in Richtung der ersten Stellung.

7. Applikator nach einem der Ansprüche 3 bis 5, **dadurch gekennzeichnet, dass** der Verschiebemechanismus eine Vorspanneinrichtung (75) aufweist zum Vorspannen der beweglichen Hülse in Richtung der zweiten Stellung.

8. Applikator nach einem der Ansprüche 1 bis 5, **dadurch gekennzeichnet, dass** der Verschiebemechanismus einen Motor (51) zum Bewegen der beweglichen Hülse (14) enthält.

9. Applikator nach einem der Ansprüche 1 bis 8, **dadurch gekennzeichnet, dass** die mindestens drei Arme einen ersten Arm (12), einen zweiten Arm (46), der sich über eine geringere Länge in der axialen Richtung erstreckt als der erste Arm (12) und ausgebildet ist zum Positionieren auf der Stabstruktur (20, 20a), enthält.

10. Applikator nach einem der Ansprüche 1 bis 8, **dadurch gekennzeichnet, dass** die mindestens drei Arme einen ersten Arm (12) und einen zweiten Arm (46) enthalten, angeordnet zum Erstrecken über eine größere Länge in der axialen Richtung als der erste Arm (12), um ein unabsichtliches Verlorengehen der Ringanordnung (21, 22) zu verhindern.

11. Applikator nach einem der Ansprüche 1 bis 10, **dadurch gekennzeichnet, dass** jeder der mindestens drei Arme (12) weiterhin einen Umfangsvorsprung (12e) aufweist, angeordnet außerhalb des Aufnahmeabschnitts (12c), um zu verhindern, dass die Ringanordnung (21, 22) aus dem Aufnahmeabschnitt (12c) herausfällt.

12. Applikator nach einem der Ansprüche 1 bis 11, weiterhin **gekennzeichnet durch** einen Rampenfläche (12d), angeordnet im Inneren der Arme (12) und auf einer Seite des hinteren Endabschnitts bezüglich des Aufnahmeabschnitts (12c), geneigt relativ zu der beweglichen Hülse (14), von der Stirnfläche (14a) der beweglichen Hülse (14) gedrückt, wenn die bewegliche Hülse (14) sich in der axialen Richtung bewegt, um eine Schwenkbewegung der Arme (12) in die Offenstellung zu erleichtern.

13. Applikator nach einem der Ansprüche 1 bis 12, weiterhin **gekennzeichnet durch** mindestens drei Aufnahmenuten (30), die in der beweglichen Hülse (14) ausgebildet sind, wobei die mindestens drei Arme (12) sich in und aus die/den Aufnahmenuten (30) bewegen, wenn sie zwischen der Offen- und der Schließstellung verschwenkt werden.

14. Applikator nach einem der Ansprüche 1 bis 13, **dadurch gekennzeichnet, dass** die Trägereinrichtung eine Arm-Haltehülse (13) enthält, die außerhalb der beweglichen Hülse (14) festgelegt ist, um die mindestens drei Arme (12) verschwenkbar zu lagern.

## Revendications

1. Applicateur destiné à monter un dispositif à bague résiliente (21, 22) autour d'un dispositif à tige (20, 20a) par dilatation radiale, comprenant :
un manchon mobile (14, 61, 84) destiné à recevoir l'entrée dudit dispositif à tige ;
un moyen de support (11, 13, 62, 69, 83, 92) destiné à supporter ledit manchon flexible de manière mobile ;
au moins trois dispositifs à bras (12, 46, 47, 63, 86), présentant une partie d'extrémité arrière (12a), agencés autour dudit manchon mobile de manière circonférentielle, destinés à s'étendre dans ladite direction axiale, ladite partie d'extrémité arrière étant sécurisée sur ledit moyen de support dans une direction radiale (A1) dudit manchon mobile ;
une partie de réception (12c, 46c, 63c, 86c), formée avec une partie d'extrémité avant desdits dispositifs à bras, destinée à recevoir un bord intérieur dudit dispositif à bague, et à se déplacer vers des positions d'état ouvert et fermé suite à un mouvement pivotant desdits dispositifs à bras, ladite partie de réception, lorsqu'elle est dans ladite position d'état fermé, étant apte à régler ledit dispositif à bague sur celle-ci, et lorsqu'elle est dans ladite position d'état ouvert, étant à apte à régler ledit dispositif à bague autour dudit dispositif à tige ;
un mécanisme de décalage (15, 50, 70, 82, 84) destiné à décaler ledit manchon mobile par rapport audit moyen de support, afin de déplacer lesdits dispositifs à bras en faisant venir en prise ledit manchon mobile avec lesdits dispositifs à bras, afin de déplacer ladite partie de réception de ladite position d'état fermé à ladite position d'état ouvert,
**caractérisé en ce que** le moyen de support supporte le manchon mobile de manière mobile dans une direction axiale (A3, A4), **en ce que** la partie d'extrémité arrière est sécurisée sur le moyen de support de manière mobile et pivotante, **en ce que** le mécanisme de décalage est apte à décaler le manchon mobile de manière à déplacer les dispositifs à bras par pivotement et à faire avancer ledit dispositif à bague à partir de ladite partie de réception avec une surface d'extrémité dudit manchon mobile (14, 61, 84).

2. Applicateur selon la revendication 1, **caractérisé en ce que** chacun desdits au moins trois dispositifs à bras (12, 46, 47, 63, 86) inclut une surface intérieure disposée de manière à s'étendre le long dudit manchon mobile lorsque ladite partie de réception se trouve dans ladite position d'état ouvert.

3. Applicateur selon la revendication 1 ou 2, **caractérisé en ce que** ledit mécanisme de décalage (15, 50, 70, 82, 94) déplace ledit manchon mobile (14, 61, 84) entre des première et seconde positions par rapport audit moyen de support (11, 13, 62, 83, 92), et ladite partie de réception (12c, 46c, 63c, 86c), lorsque ledit manchon mobile se trouve dans ladite première position, se trouve ladite position d'état fermé, et lorsque ledit manchon mobile (14, 61, 84) se trouve dans ladite seconde position, se trouve dans ladite position d'état ouvert.

4. Applicateur selon l'une quelconque des revendications 1 à 3, **caractérisé en ce que** ledit mécanisme de décalage inclut :
une partie de préhension (11a, 69, 83, 92) sur laquelle est sécurisé ledit moyen de préhension ;
un levier de décalage (15, 70, 82, 94) disposé sur ladite partie de préhension (11a) d'une manière mobile et pivotante ;
un bras de levier d'entrée (15b) disposé sur une première extrémité dudit levier de décalage (15), et
un bras de levier de sortie (15a) disposé sur une seconde extrémité dudit levier de décalage (15), en vue d'une prise avec ledit manchon mobile (14, 61, 84).

5. Applicateur selon l'une quelconque des revendications 1 à 4, **caractérisé en ce que** qu'il comprend en outre une saillie à came (63a), formée au niveau de ladite partie d'extrémité arrière desdits au moins trois dispositifs à bras (63), destinée à venir en contact avec ledit manchon mobile (61) afin d'empêcher un mouvement pivotant,
dans lequel ledit manchon mobile (61) inclut une saillie de prise (61c) destinée à une prise avec ladite saillie à came (63a) dans ladite première position, afin de régler ladite partie de réception (63c) dans ladite position d'état fermé.

6. Applicateur selon l'une quelconque des revendications 3 à 5, **caractérisé en ce qu'**il comprend en outre un premier dispositif de sollicitation (16, 81) destiné à solliciter ladite partie de réception (63c) vers ladite position d'état fermé ;
dans lequel ledit mécanisme de décalage (70) inclut un second dispositif de sollicitation (43) destiné à solliciter ledit manchon mobile (61) vers ladite première position.

7. Applicateur selon l'une quelconque des revendications 3 à 5, **caractérisé en ce que** ledit mécanisme de décalage inclut un dispositif de sollicitation (75) destiné à solliciter ledit manchon mobile (61) vers ladite seconde position.

8. Applicateur selon l'une quelconque des revendications 1 à 5, **caractérisé en ce que** ledit mécanisme de décalage inclut un moteur (51) destiné à déplacer ledit manchon mobile (14).

9. Applicateur selon l'une quelconque des revendications 1 à 8, **caractérisé en ce que** lesdits au moins trois dispositifs à bras incluent un premier dispositif à bras (12), et un deuxième dispositif à bras (46), disposés afin de s'étendre avec une longueur dans ladite direction axiale plus petite que ledit premier dispositif à bras (12), et apte à un positionnement sur ledit dispositif à tige (20, 20a).

10. Applicateur selon l'une quelconque des revendications 1 à 8, **caractérisé en ce que** lesdits au moins trois dispositifs à bras incluent un premier dispositif à bras (12), et un deuxième dispositif à bras (46), disposés afin de s'étendre avec une longueur dans ladite direction axiale plus grande que ledit premier dispositif à bras (12), afin d'empêcher toute perte accidentelle dudit dispositif à bague (21, 22).

11. Applicateur selon l'une quelconque des revendications 1 à 10, **caractérisé en ce que** chacun desdits au moins trois dispositifs à bras (12) inclut en outre une saillie périphérique (12e), disposée à l'extérieur de ladite partie de réception (12c), afin d'empêcher ledit dispositif à bague (21, 22) de sortir de ladite partie de réception (12c).

12. Applicateur selon l'une quelconque des revendications 1 à 11, **caractérisé en ce qu'**il comprend en outre une surface à gradins (12d), disposée à l'intérieur desdits dispositifs à bras (12) et sur un côté de ladite partie d'extrémité arrière à partir de ladite partie de réception (12c), inclinée par rapport audit manchon mobile (14), poussée par ladite surface d'extrémité (14a) dudit manchon mobile (14) lorsque ledit manchon mobile (14) se déplace dans ladite direction axiale, afin de faciliter un mouvement pivotant desdits dispositifs à bras (12) vers ladite position d'état ouvert.

13. Applicateur selon l'une quelconque des revendications 1 à 12, **caractérisé en ce qu'**il comprend en outre au moins trois rainures de réception (30) formées dans ledit manchon mobile (14), lesdits au moins trois dispositifs à bras (12) entrant respectivement dans lesdites rainures de réception (30), et sortant de celles-ci, lorsqu'ils sont déplacés par pivotement entre lesdites positions d'état ouvert et fermé.

14. Applicateur selon l'une quelconque des revendications 1 à 13, **caractérisé en ce que** ledit moyen de support inclut un manchon de support de bras (13) sécurisé à l'extérieur dudit manchon mobile (14), en vue de supporter lesdits au moins trois dispositifs à bras (12) d'une manière mobile et pivotante.
